# EUROPEAN PATENT SPECIFICATION

(11) **EP 1 477 102 B1**
(45) Date of publication and mention of the grant of the patent: **10.05.2006**
(21) Application number: 04017823.8
(22) Date of filing: 28.07.2004
(51) Int. Cl.: A61B 1/00

(54) **Structure for probe insertion**
Vorrichtung zum Einfügen einer Sonde
Structure pour l'introduction d'une sonde

(43) Date of publication of application: 17.11.2004
(73) Proprietor: PHONAK AG, 8712 Stäfa (CH)
(72) Inventor: Bächler, Herbert, 8706 Meilen (CH); Berg, Christian, 8713 Uerikon (CH)
(74) Representative: Troesch Scheidegger Werner AG

(56) References cited:
- US-A- 4 207 872
- US-A- 4 577 621
- US-A1- 2003 045 778
- US-B1- 6 293 908
- US-B1- 6 626 826

## Description

The present invention refers to an insert structure for insertion into an orifice, a canal, a tube, etc. of a human or animal body such as an ear canal, an intestinal tract, a gullet, etc. according to the wording of claim 1 and a method of introducing a sensor, a scanning device, a video head and the like into an orifice, a canal, a tube, etc. of a human or an animal creature.

For a proper fit e.g. of a hearing device it has always been considered vital to obtain good data of the ear canal. In case of hearing instruments for hard of hearing patients the data needs to cover a significant part of the canal and preferably beyond the "second bend". Traditionally, data has been obtained with a silicon ear impression and this process is well-established. Being a manual and quite difficult process, alternative technologies have been searched for a long time and currently the focus is on direct scanning of the ear. An intermediate stage is scanning of ear impressions for computer-based in-the-ear (ITE) shell or behind-the-ear (BTE) mold production.

The pursuit of direct ear scanning is still ongoing and there are several approaches being studied. A number of problems remain to be solved, one being the way to insert the scanning head (probe) into the ear canal without hurting the patient, changing the shape of the canal or impacting the data acquisition. This is one of the subjects of the present invention.

In this respect in WO 02/091920 A1 the insertion of a scanning probe into an ear canal is proposed. Therefore, this prior art document is highly relevant and interesting, but does not give any contribution to the above mentioned problem.

In WO 02/16867 A1 a 360° probe-shaped non-contact scanning device is described. It also includes the post-processing of the data to obtain high-precision 3D data for individual ear canal shapes. But again nothing in this prior art document is addressing to bend within the canal and insertion depth control.

In WO 02/16865 A2 a calibration method for ear scanning probes in order to achieve needed precision is described. The invention itself again does not touch the topic of the present invention.

The US 4 577 621 describes an endoscope comprising two flexible tube members, each having a bellows like expansion and contracting member which are expandable or contractible to allow alternate movement of the two tube members into a body portion of a person.

Document US 4 207 872 discloses a device for assisting in the advancement of an endoscope through the colon comprising a hollow tube and a plurality of protrusions expanding outwardly forming part of an inflatable chamber and therefore connected among them.

There are several still open issues and real problems pursuing replacement of traditional ear impression taking by "direct ear scanning" one way or another. Most approaches involve the use of light (LED, laser, etc.); some propose video, ultrasound, computer tomography and even MRI. Challenges relate to issues like: resolution, physical reference points, ear canal skin surface condition, post-processing of 3D information and touchless insertion/extraction of probes. The latter problem has not really been solved by any significant patent documents or publications to date and remains an obstacle in obtaining good 3D data of the undistorted ear canal. In addition, there are problems like skin scratches during insertion and extraction and even more severe: harm to the tympanic membrane, which is both very unpleasant and bears the risk of a distorted tympanic membrane function (e.g. conductive hearing loss).

Besides the described problem in relation particularly to insertion into the ear canal, this of course is a general problem in relation to the insertion of any kind of scanning probes, sensors, video heads, tubes like cannulas or catheters etc. into orifices, canals, tubes and the like of the human or animal body. Besides the ear canal this could be e.g. the intestinal tract, the gullet, etc.

It is therefore a subject of the present invention to propose a solution for introducing in an easy and reliable manner a device such as a scanning device, a sensor, a cannula or catheter, a testing probe, a video head, etc. into an orifice, a canal, a tube, etc. of a human or animal body without causing skin scratching, injuring, harming, etc. of tissues, cartilage or bone skin etc.

According to the present invention an insert structure is proposed according to the wording of claim 1. The inventive insert structure for insertion into an orifice, a canal, a tube, etc. of the human or animal body or creature respectively such as an ear canal, an intestinal tract, a gullet of a human or animal being comprises at least one longitudinally extending insert member and a plurality of at least nearly perpendicularly extending further members placed longitudinally at a distance in between the further members along the insert member, the further members being at least of a flexible and/or soft material.

According to a further design the insert member is a tube-or rod-like flexible member.

Again according to a further design it is proposed that the insert member being a tube-like member open at both ends, such that the tube acts like a guidance or supporting structure for the introduction of a sensor, a viewing probe, a scanning probe, a video head, a cannula or catheter, etc. being inserted into the canal or tube of the human or animal being through the tube-like member.

If e.g. optical scanning, viewing or video scanning is planned, it is preferred that the tube-like insert member is transparent, so that viewing, scanning etc. through the tube wall is possible.

Furthermore, according to a further design it is proposed that the at least nearly perpendicularly extending further members are arranged with equal spacing between each other.

Further preferred designs are described in the dependent claims.

Furthermore, a method for introducing a sensor, a scanning probe, a video scanning device, a cannula or catheter, etc. into an orifice, a canal, a tube, etc. of a human or animal body is proposed according to the wording of claim 10. The sensor, the scanning device, etc. can be introduced by using an insert structure as proposed above having a tube-like insert member being open at least on one end. The e.g. pin-like sensor or scanning device can be introduced into the orifice of the tube-like insert member and can be moved within the ear canal or tube of the human or animal body either by moving the insert structure into the canal or tube of the body and/or by moving the sensor within the tube-like insert member.

Again, further preferred methods are described within the dependent claims.

The invention will now be explained in more detail based on design examples and with reference to the attached drawings, in which:
- Fig. 1: shows schematically and in perspective view an inventive insert structure;
- Fig. 2: shows the insert structure of fig. 1 in a longitudinal section view, and
- Fig. 3: shows schematically the insertion of an inventive insert structure into an ear canal in a longitudinal section view.

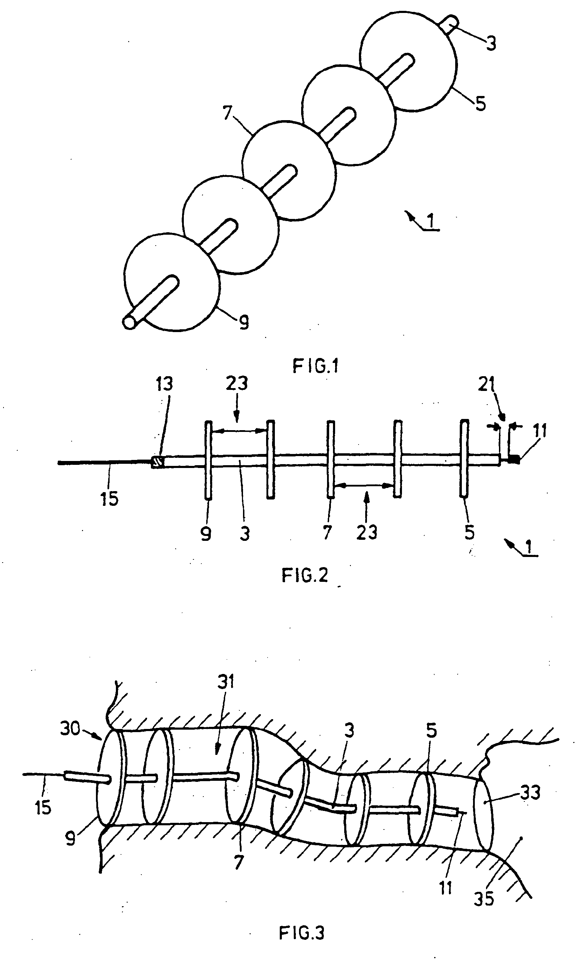

Fig. 1 shows schematically and in lengthwise perspective view an inventive insert structure 1 mainly comprising a central tube- or rod-like insert member 3, which preferably has to be very flexible. Along the insert member 3 various at least nearly perpendicularly extending further members or disk-like members 5 are arranged, whereby the distance between the various disk-like further members 5 preferably is approx. equal.

It is preferred that at least some of the perpendicularly extending further members or disk-like members are specially marked, like e.g. a first bend marker 7 and a second bend marker 9. Using marked disk-like members it is possible to know at any time the depth of the insertion of the insert member into e.g. an ear canal. Standard markers will give a good indication of the depth of the introduction into the ear canal.

In fig. 2 the insertion structure 1 of fig. 1 is shown in a longitudinal section view in more detail. Again, the central insert member 3 is shown, which is preferably a tube with open ends on both sides. Therefore, e.g. an optical sensor probe, such as an optical fiber, is introduced through the tube-like insert member until the tip of the fiber slightly extends beyond the front end of the insert member by a very short distance 21. In case of an ear canal, this distance may be e.g. approx. 2mm. Such a tip on an optical scanning probe is shown and designated with the reference no. 11 in fig. 2. In addition again the disk-like members 5, 7 and 9 are shown. The disks are arranged, each at a distance 23 from the next disk-like member, whereby the distance 23 in case e.g. of an ear canal may be approx. 5 mm.

At the back end of the insert member 3 e.g. an electric wire, an optical fiber, etc. 15 can be introduced and be connected through the tube-like insert member 3 with the tip 11 of the optical scanning probe or optical fiber, respectively, extending at the front end of the tube-like insert member.

As already explained in relation to fig. 1 it is very important always to know the depth of the introduction of the insertion structure into e.g. the ear canal, so that there is no danger of injuring the tympanic membrane or tympanum respectively. Therefore, again in fig. 2 e.g. the disk-like members 7 and 9 are especially marked.

In order to ensure that an inserted scanning probe 11 does not extend beyond the front end of the insert member 3 by more than a certain predetermined distance 21, a marker 13 can be arranged on the electric wire, optical fiber, etc. 15. This prevents the scanning probe 11 from being inserted too far into e.g. the ear canal, and therefore safeguards the tympanic membrane or tympanum respectively from being injured. The marker 13 can also be a mechanical stopper which is securely attached to the electric wire, optical fiber, etc. 15.

In fig. 3 it shall now be shown in more detail and based on an example how the insertion structure shall be introduced into e.g. an ear canal.

Fig. 3 shows schematically and in a longitudinal section view an ear canal 31 extending from the ear opening 30 to the tympanic membrane 33 which separates the ear canal 31 from the middle-ear 35.

Looking at fig. 3 it becomes very obvious that the contours of the ear canal wall are quite complex and furthermore it has to be understood that these contours differ very substantially from one ear canal to another. In other words, an ear canal structure is very individual and the structures may differ quite dramatically from one human or animal being to another.

Therefore, when introducing any kind of a sensor, a scanning device and the like it is very difficult to introduce such a device without injuring or harming the internal skin of the ear canal. Furthermore, it is important that the introduction of such a device is stopped before it reaches the tympanic membrane 33. Both problems, which mean injuring the skin of the ear canal as well as safely inserting the scanning probe through the tube-like insert member 3, are solved by using the inventive insert structure since e.g. the scanning head can never come into contact with the skin of the ear canal. Furthermore, by using marked disk-like members 7 and 9 and/or by using marks on the tube-like insert member one always knows the depth of the insertion of the insert structure. Furthermore, due to the marker 13 arranged on the electric wire, optical fiber, etc. 15 one always knows when the scanning probe which is inserted through the insert tube has reached the front end of the tube-like insert member and one can stop the further insertion of the device.

A further advantage of the inventive structure is that the tube-like member is more or less within the center axis of the ear canal and any kind of scanning of the ear canal structure can be executed in the more or less optimal position of the optical scanning probe, the video camera, the sensor, etc.

But of course, the invention is not at all limited to the introduction of scanning probes or sensors as described above, but it is also possible to introduce cannulas or catheters into at least a first section of the intestinal tract or into the gullet, etc. Also the introduction of cannulas or catheters may cause harm to the skin, e.g. of the intestinal tract, of the gullet and the like.

The flexibility either of the insert member as well as of the disk-like members may be of great importance. Preferably, both members are made out of a flexible material such as e.g. an elastomeric polymer, rubber, silicon rubber, at least partially foamed material, etc. Of course any kind of suitable e.g. polymeric material may be used, the important point is that the material has a high compatibility to skin, is acid resistant and resistant against water. And of course the material should not be toxic even at a very low level.

Furthermore, instead of using the disc-like members, it is also possible to use star-like members or parts comprising perpendicularly extending fingers also made out of an elastomeric polymer, the elastic fingers sliding along the interior wall of the ear canal during the introduction of the insert structure. In that sense it is possible to use any kind spacing members to guide the insert member into the canal or tube, such as e.g. the ear canal.

In summary, the idea of the present invention is to propose a structure that will address and solve the following problems:
- Insertion and extraction of e.g. an ear scanner probe without touching the ear canal independent of the canal shape,
- insertion depth control. There are indicators on the insertion support structure indicating e.g. by color coding insertion depth. E.g. ear canal depth is on average about 28 mm from the first bend with a standard deviation of about 2.6 mm. From the second bend in the canal the average is about 17 mm with a similar standard deviation. Hence there will be no risk to affect the tympanic membrane if the probe extends e.g. 2 mm from the front end of the supporting structure. In addition the probe itself could contain viewing properties such as in case e.g. of a video head, an optical scanner, etc.

Furthermore, an optional solution could be to make the supporting structure transparent to allow recording while pulling the probe out through the tube.

A further solution could be to apply a flexible and possibly disposable membrane or envelope over the whole supporting or insert structure. This membrane would be slightly pressed towards e.g. the ear canal by the disk-like members such as e.g. the silicon rings on the central insert tube. The probe would then record the membrane shape, which may be provided with a specific pattern and which is prepared to allow optimal e.g. optical scanning. The offset or the difference to the real canal wall data can be corrected by geometric (e.g. thickness of the membrane) and statistical data.

## Claims

1. Insert structure for insertion into an orifice, a canal, a tube and the like of a human or animal creature such as an ear canal, an intestinal tract, a gullet, etc. comprising at least one longitudinally extending insert member (3) and a plurality of at least nearly perpendicularly extending further members (5, 7, 9) placed longitudinally at a distance to each other along the insert member and only connected to each other by the insert member, the further members (5, 7, 9) being at least of a flexible and/or soft material.

2. Insert structure according to claim 1, the insert member (3) being a rod- or tube-like flexible member.

3. Insert structure according to one of the claims 1 or 2, the insert member being a tube-like member open at least on one end, such that the tube-like member acts as a guidance or supporting structure for a sensor, a viewing probe, a scanning probe, etc. being inserted into the orifice, the canal, the tube through the tube-like insert member.

4. Insert structure according to one of the claims 1 to 3, the tube-like member being transparent such that scanning, viewing, visual scanning by video, etc. through the tube wall is possible.

5. Insert structure according to one of the claims 1 to 4, the at least nearly perpendicularly extending further members (5, 7, 9) are arranged at equivalent distances along the insert member.

6. Insert structure according to one of the claims 1 to 5, **characterized in that** at the insert member indicators (13) are arranged for insertion depth control and/or that the further members (5, 7, 9) are labeled or marked such that an insertion depth control is possible.

7. Insert structure according to one of the claims 1 to 6, **characterized in that** the further members are disk-like flexible and/or soft members keeping the insert member at a distance from the internal wall of the orifice, the canal, the tube such as e.g. the ear canal wall.

8. Insert structure according to one of the claims 1 to 7, **characterized in that** the further members consist of an elastomeric and/or rubber-like or foam-like polymeric material.

9. Insert structure according to one of the claims 1 to 8, **characterized in that** over the outside surface of the insert structure a membrane-like preferably highly flexible envelope is arranged, e.g. provided with a pattern like structure for allowing e.g. optical scanning.

10. Insert structure according to one of the claims 1 to 9 for introducing a sensor, a scanning probe, a video scanning device, etc. into an orifice, a canal, a tube, etc. or a human or animal body, **characterized in that** the sensor or probe is inserted via a tube-like insert member.

11. Insert structure for the introduction according to claim 10, **characterized in that** the sensor or probe is inserted along the tube-like insert member until it reaches the front end of the tube like insert member and that the sensor or probe is moved within the orifice, canal or tube of the animal or human body by moving the insert structure and/or by moving the sensor or probe respectively along the tube-like insert member.

## Patentansprüche

1. Anordnung für das Einführen in eine Öffnung, einen Kanal, eine Röhre oder dergleichen einer menschlichen oder tierischen Kreatur wie in einen Ohrkanal, den Darmtrakt, die Speiseröhre etc., aufweisend mindestens ein sich längs erstreckendes Einführteil (3) und eine Vielzahl sich wenigstens nahezu senkrecht erstreckender weiterer Teile (5, 7, 9), welche in Längsrichtung zueinander beabstandet entlang des Einführteils angeordnet sind, wobei sie miteinander lediglich über das Einführteil verbunden sind und wobei die weiteren Teile (5, 7, 9) mindestens aus einem flexiblen oder weichen Material bestehen.

2. Einführanordnung nach Anspruch 1, wobei das Einführteil (3) ein stab- oder rohrartiges flexibles Teil ist.

3. Einführanordnung nach einem der Ansprüche 1 oder 2, wobei das Einführteil ein rohrartiges Teil ist, welches mindestens an einem Ende offen ist, derart, dass das rohrartige Teil als Führungs- oder Unterstützungsanordnung für einen Sensor, eine Betrachtungssonde, eine Abtastsonde etc. dient, welcher bzw. welche in die Öffnung, den Kanal, die Röhre durch das rohrartige Einführteil eingeführt wird.

4. Einführanordnung nach einem der Ansprüche 1 - 3, **dadurch gekennzeichnet, dass** das rohrartige Teil transparent ist, derart, dass Abtasten, Betrachten, visuelles Abtasten mittels Video etc. durch die Rohrwandung möglich ist.

5. Einführanordnung nach einem der Ansprüche 1 - 4, **dadurch gekennzeichnet, dass** die wenigstens nahezu sich senkrecht erstreckenden weiteren Teile (5, 7, 9) mit gleich bleibender Distanz entlang des Einführteiles angeordnet sind.

6. Einführanordnung nach einem der Ansprüche 1 - 5, **dadurch gekennzeichnet, dass** am Einführteil Anzeigen (13) angeordnet sind für die Steuerung der Einführtiefe und/oder dass die weiteren Teile (5, 7, 9) **gekennzeichnet** oder markiert sind, derart, dass eine Steuerung der Einführtiefe möglich ist.

7. Einführanordnung nach einem der Ansprüche 1 - 6, **dadurch gekennzeichnet, dass** die weiteren Teile scheibenartige, flexible und/oder weiche Teile sind, welche das Einführteil auf Distanz von der inneren Wandung der Öffnung des Kanals oder der Röhre halten, wie beispielsweise der Ohrkanalwandung.

8. Einführanordnung nach einem der Ansprüche 1 - 7, **dadurch gekennzeichnet, dass** die weiteren Teile aus einem elastomeren und/oder gummiartigen oder schaumartigen Polymermaterial bestehen.

9. Einführanordnung nach einem der Ansprüche 1 - 8, **dadurch gekennzeichnet, dass** über die äussere Oberfläche der Einführanordnung eine membranartige, vorzugsweise hochflexible Hülle angeordnet ist, beispielsweise versehen mit einer rasterartigen Struktur für das Ermöglichen beispielsweise von optischem Abtasten.

10. Einführanordnung nach einem der Ansprüche 1 - 9 für das Einführen eines Sensors, einer Abtastsonde, einer Videoabtasteinrichtung etc. in eine Öffnung, einen Kanal, eine Röhre etc. oder einen menschlichen oder tierischen Körper, **dadurch gekennzeichnet, dass** der Sensor oder die Sonde durch das rohrartige Einführteil eingeführt ist.

11. Einführanordnung für das Einführen nach Anspruch 10, **dadurch gekennzeichnet, dass** der Sensor oder die Sonde entlang dem rohrartigen Einführteil eingeführt wird, bis das Frontende des rohrartigen Einführteiles erreicht wird und dass der Sensor oder die Sonde innerhalb der Öffnung des Kanals oder des Rohres des menschlichen oder tierischen Körpers bewegt wird durch Bewegen der Einführanordnung und/oder durch Bewegen des Sensors respektive der Sonde entlang des rohrartigen Einführteils.

## Revendications

1. Structure d'insertion destinée à être introduite dans un orifice, un canal, un tube et similaire d'une créature humaine ou animale, comme dans un canal auditif, un tract intestinal, un oesophage etc., comprenant au moins un élément d'insertion (3) s'étendant longitudinalement et une multitude d'autres éléments (5, 7, 9) s'étendant au moins approximativement perpendiculairement et placés espacés l'un par rapport à l'autre le long de l'élément d'insertion et reliés entre eux uniquement par ledit élément d'insertion, lesdits autres éléments (5, 7, 9) étant au moins en un matériau flexible et/ou mou.

2. Structure d'insertion selon la revendication 1, ledit élément d'insertion (3) étant un élément flexible en forme de barre et/ou tube.

3. Structure d'insertion selon l'une des revendications 1 ou 2, ledit élément d'insertion étant un élément tubulaire ouvert à au moins une extrémité, de manière à ce que l'élément tubulaire agisse comme guide ou support pour un détecteur, un capteur d'images, un balayeur, etc., inséré dans l'orifice, le canal ou le tube à travers ledit élément d'insertion tubulaire.

4. Structure d'insertion selon l'une des revendications 1 à 3, ledit élément tubulaire étant transparent tel que le balayage, la prise d'image, le balayage visuel par vidéo etc. à travers la paroi du tube soit possible.

5. Structure d'insertion selon l'une des revendications 1 à 4, lesdits autres éléments (5, 7, 9) qui s'étendent au moins approximativement perpendiculairement étant disposés à distances équivalentes le long de l'élément d'insertion.

6. Structure d'insertion selon l'une des revendications 1 à 5, **caractérisée en ce que** des indicateurs (13) sont prévus sur l'élément d'insertion pour le contrôle de la profondeur d'insertion et/ou **en ce que** lesdits autres éléments (5, 7, 9) portent des marques de manière à permettre le contrôle de la profondeur d'insertion.

7. Structure d'insertion selon l'une des revendications 1 à 6, **caractérisée en ce que** lesdits autres éléments sont des éléments en forme de disque flexible et/ou mou qui maintiennent l'élément d'insertion à distance de la paroi interne de l'orifice, du canal, du tube, comme par exemple de la paroi du canal auditif.

8. Structure d'insertion selon l'une des revendications 1 à 7, **caractérisée en ce que** lesdits autres éléments sont en un matériau élastomère et/ou de gomme élastique ou mousse polymère.

9. Structure d'insertion selon l'une des revendications 1 à 8, **caractérisée en ce qu'**une enveloppe sous forme de membrane, de préférence à haute flexibilité, est disposée sur la surface extérieure de la structure d'insertion, p.ex. munie d'un dessin structuré pour permettre p.ex. un balayage optique.

10. Structure d'insertion selon l'une des revendications 1 à 9, pour introduire un détecteur, un capteur balayeur, un balayeur vidéo etc. dans un orifice, un canal, un tube etc. d'un corps humain ou animal, **caractérisée en ce que** le détecteur ou capteur est inséré via un élément d'insertion tubulaire.

11. Structure d'insertion pour l'insertion selon la revendication 10, **caractérisée en ce que** le détecteur ou le capteur est inséré le long de l'élément d'insertion tubulaire jusqu'à atteindre l'extrémité avant de l'élément d'insertion tubulaire et **en ce que** le détecteur ou le capteur est déplacé dans l'orifice, le canal ou le tube du corps animal ou humain par déplacement de la structure d'insertion et/ou par déplacement du détecteur ou du capteur le long dudit élément d'insertion tubulaire.
